# EUROPÄISCHE PATENTSCHRIFT

(11) **EP 2 099 370 B1**
(45) Veröffentlichungstag und Bekanntmachung des Hinweises auf die Patenterteilung: **20.02.2019**
(21) Anmeldenummer: 07847010.1
(22) Anmeldetag: 05.12.2007
(51) Int. Cl.: A61L 29/06, A61B 17/00, A61L 29/08, A61L 29/16, A61J 15/00, A61B 17/34

(54) **SCHLAUCH FÜR DIE ENTERALE ERNÄHRUNG**
TUBE FOR ENTERAL NUTRITION
TUBULURE DE NUTRITION ENTÉRALE

(30) Priorität: 11.12.2006 EP 06025588
(43) Veröffentlichungstag der Anmeldung: 16.09.2009
(73) Patentinhaber: N.V. Nutricia, 2712 HM Zoetermeer (NL)
(72) Erfinder: WOLKENSTÖRFER, Reinhold, 91077 Neunkirchen (DE); HORBACH, Thomas, 91052 Erlangen (DE)
(74) Vertreter: von Hellfeld, Axel
(86) Internationale Anmeldenummer: PCT/EP2007/010582
(87) Internationale Veröffentlichungsnummer: WO 2008/071330

(56) Entgegenhaltungen:
- WO-A-95/04564
- WO-A-03/010354
- US-A- 3 070 132
- US-A- 4 105 732
- US-A- 5 837 275
- US-A- 6 060 000
- US-A1- 2001 051 669
- US-A1- 2004 220 534
- US-B1- 6 530 951

## Beschreibung

Die Erfindung betrifft einen Schlauch für die enterale Ernährung, insbesondere einen PEG-Schlauch (PEG = Perkutane Endoskopische Gastrostomie)
Die perkutane endoskopische Gastrostomie ist die Anlage einer Ernährungssonde mit Hilfe eines Endoskopes durch die Bauchwand in den Magen. Hierfür wird regelmässig ein Gastroskop eingesetzt, also ein biegsames optisches Instrument mit Objektiv und Okular oder einer elektronischen Bildübertragungseinrichtung. Ein PEG-Schlauch (auch PEG-Sonde genannt), dient der enteralen Ernährung, also einer Ernährung direkt über den Magen-Darm-Trakt. Über den PEG-Schlauch kann Flüssigkeit bzw. flüssige oder dünnbreiige Nahrung verabreicht werden.

Der PEG-Schlauch ist eines der häufigst eingesetzten Mittel zur langfristigen enteralen Ernährung von Patienten, die aufgrund von bestimmten Erkrankungen nicht mehr ausreichend Nahrung über den Mund zu sich nehmen können.

In der Regel erfolgt die Positionierung eines PEG-Schlauches mit Hilfe der sogenannten Fadendurchzugsmethode. Dabei schiebt der Arzt ein Gastroskop über Mund und Speiseröhre in den Magen. Durch Einblasen von Luft entfaltet sich der Magen, sodass er besser einsehbar ist. Sodann wird eine Hohlnadel durch die Bauchwand unter endoskopischer Kontrolle durch die Bauchdecke in den Magen eingeschoben. In der Hohlnadel steckt ein Kunststoffröhrchen. Sodann wird die Hohlnadel herausgezogen und durch das verbleibende Kunststoffröhrchen wird ein Faden in den Magen eingeführt. Dieser Faden kann dann mit einer Zange erfasst werden, die der Arzt über das Gastroskop eingeführt hat. Faden, Zange und Gastroskop werden dann über die Speiseröhre und den Mund herausgezogen. Der PEG-Schlauch kann dann mit dem aus dem Mund herausragenden Fadenende verbunden werden und durch Zug am anderen Fadenende wird die Sonde über Mund und Speiseröhre in den Magen gebracht und teilweise durch die Bauchdecke gezogen. Platten an dem PEG-Schlauch positionieren ihn in Bezug auf die Bauchdecke und den Magen. Sodann kann das herausragende Ende des PEG-Schlauches mit Mitteln zum Zuführen von Nahrung verbunden werden.

Zwar hat sich die perkutane endoskopische Gastrostomie weithin durchgesetzt, jedoch besteht nach wie vor seit langem ein erhebliches Infektionsrisiko, das in den zwei-stelligen Prozentbereich reichen kann. So wird noch im Jahre 2000 allein für die peristaltische Infektion ein Prozentsatz von 5-15% berichtet (C.H. Löser, DMW 125, 805). Insbesondere treten Nekrose und Mykose auf.

Der Stand der Technik kennt verschiedene Versuche, durch PEG-Schläuche verursachte Infektionen zu verhindern. So werden möglichst glatte Oberflächen am PEG-Schlauch eingesetzt, auch hydrophile Oberflächen, sowie Hydrogele, Heparin und Heparioide. Auch monoklonale Antikörper werden bei Kathetern eingesetzt. Studien liegen vor zur Verhinderung von Infektionen bei der PEG mittels Antibiotika.

Allerdings haben Antibiotika auch Nachteile, insbesondere kann die antimikrobielle Wirkung schon nach 24 Stunden nachlassen und die Wirkung ist nicht lokal begrenzt. Auch das Anwendungsspektrum ist begrenzt und der Patient kann Resistenz entwickeln. Auch unter Kostengesichtspunkten sind Antibiotika nachteilig.

Die US 2004/0220534 A1 beschreibt insbesondere eine Magensonde mit einer Wand aus einem hydrophoben Polymer, wobei auf einer Außenfläche der Wand ein hydrophiles Polymer aufgetragen ist mit einer anti-mikrobiellen Verbindung, bestehend aus einem phosphor-basierten Glass, in dem ein Metall dispergiert ist, insbesondere Silber-Ionen. Eine solche Lehre ist nicht Gegenstand der vorliegenden Erfindung.

Die US 6,060,000 beschreibt insbesondere Katheter unter Verwendung der oligodynamischen Iontophorese. Silber-Ionen werden zur Erzielung eines anti-bakteriellen Effekts mit Hilfe eines elektrischen Potentials in die umgebende Flüssigkeit abgegeben. Die Wanderung des Katheters enthält abgegrenzte Bereiche, zum Beispiel in Form
langgestreckter Streifen, die Elektroden zur Erzeugung des genannten elektrischen Potentials enthalten.

Die WO 95/04564 beschreibt eine enterale Ernährungssonde mit einer Außenfläche, die mit Parylene beschichtet ist.

Die US 2001/0051669 A1 beschreibt medizinische Einrichtungen, wie insbesondere Katheter oder dergleichen, mit einer Gleitschicht zur Reduzierung von Reibung.

Ferner betrifft die US 5,837,275 antimikrobielle Beschichtungen aus Metall oder Pulver, um beispielsweise einen antimikrobiellen Effekt von Metallionen zu nutzen.

Der Erfindung liegt die Aufgabe zugrunde, einen Schlauch für die enterale Ernährung bereitzustellen, bei dem mit einfachen Mitteln die Infektionsgefahr reduziert ist.

Hierfür stellt die Erfindung einen Schlauch mit einer antiseptischen und/oder antimikrobiellen Verbindung gemäß dem unabhängigen Anspruch 1 bereit. Bevorzugte Ausführungsformen sind in den abhängigen Ansprüchen definiert.

Als Verbindung hierfür sind besonders geeignet Metallsalze, insbesondere Silbersalze, die Metallionen (Silberionen) oder kolloidales Silber freisetzen.

Dabei liegt die Metallionen oder kolloidales Silber freisetzende Verbindung in Form von sogenannten Nanopartikeln vor, also Partikeln mit Abmessungen im Nano-Bereich.

Die Erfindung hat insbesondere folgende Vorteile: ein sehr breitbandiges antimikrobielles Spektrum; eine hohe Aktivität gegen sessile Organismen und Varianten in sehr kleinen Kolonien; eine langanhaltende Aktivität über Wochen, Monate oder sogar Jahre; keine Induktion von Resistenz; keine Förderung von Thrombosegefahr oder Zytotoxizität; keine Änderungen der Materialeigenschaften des Schlauchkunststoffes; sowie geringe Kosten.

Gemäß einer bevorzugten Ausgestaltung ist der Schlauch nicht vollständig in allen Bereichen mit der antiseptischen und/oder antimikrobiellen Substanz versehen, sondern nur in Teilbereichen. Diese Teilbereiche des Schlauches erstrecken sich bevorzugt streifenförmig, insbesondere in Längsrichtung des Schlauches, sodass eine einfache Herstellung mit Extrusionsverfahren ermöglicht ist. Eine bevorzugte Ausgestaltung sieht vor, dass die streifenförmigen Oberflächenbereiche zick-zack-förmig oder wellenförmig sind. Hierdurch wird eine gleichmäßig Abgabe und Verteilung der Metallionen gefördert. Auch können zu diesem Zweck streifenförmige Bereiche mit der antimikrobiellen Verbindung vorgesehen sein, die eine variierende Breite über die Längserstreckung aufweisen oder auch wendelförmig angeordnet sind.

Für eine Förderung der Inspektionsmöglichkeiten kann der Schlauch bevorzugt teilweise mit transparenten Bereichen ausgestaltet sein, die sich in Längsrichtung erstrecken, zum Beispiel indem sein Kunststoffmaterial transparent ist.

Eine andere bevorzugte Ausgestaltung der Erfindung sieht auf der Oberfläche des Schlauches eine Parylene-Beschichtung vor. Diese Parylene-Beschichtung kann auf der Außenfläche und/oder der Innenfläche des Schlauches vorgesehen sein. Auch kann zusätzlich oder alternativ zu der Parylene-Beschichtung eine Anti-Krustationsschicht vorgesehen sein.

Eine weitere bevorzugte Ausgestaltung der Erfindung sieht einen Schlauch aus zum Beispiel Polyurethan oder Silikon vor, der auf der Außenfläche und/oder der Innenfläche mit einer Parylene-Beschichtung versehen ist, wobei über der Parylene-Beschichtung eine weitere Beschichtung aus einem Metallionen freisetzenden Material aufgetragen ist. Bei den Metallionen kann es sich zum Beispiel um Silber-Ionen oder Titan-Ionen handeln. Auch kann eine weitere Beschichtung vorgesehen sein, die kolloidales Silber freisetzt.

Bevorzugt wird Parylene C eingesetzt und/oder Parylene N. Es wurde festgestellt, dass für den hier vorgesehenen Einsatz Parylene C eine besonders gute chemische Resistenz aufweist und eine leichte antibakterielle Wirkung. Parylene N hingegen zeichnet sich durch eine besondere Gleitfähigkeit aus. Geeignet ist auch ein Mix aus Parylene C und Parylene N.

Die Erfindung betrifft auch allgemein Schläuche für die enterale Ernährung, insbesondere Sonden und Katheter, die sich dadurch auszeichnen, dass sie mit einer Beschichtung aus Parylene oder Poly-Parylene versehen sind, wobei die Beschichtung Substanzen enthält, welche antiseptisch oder antimikrobiell wirken, insbesondere durch Freisetzung von Metallionen oder kolloidalem Silber

Auch hier, wie bei den anderen Ausführungsformen, insbesondere dem PEG-Schlauch, werden die antiseptisch oder antimikrobiell wirkenden Substanzen vorzugsweise in Form von Nanopartikeln eingebracht oder aufgebracht.

Die Schichtstärke der Beschichtung aus Parylene kann bei allen hier beschriebenen Ausführungsbeispielen vorzugsweise im µm-Bereich liegen, insbesondere im Bereich von 0,2 bis 10 µm, besonders bevorzugt im Bereich von 1 bis 5 µm.

Dabei kann auf die oben bereits angesprochene Parylene-Beschichtung noch eine weitere Beschichtung aufgetragen werden aus einem Material, welches Ag- oder Ti-Ionen oder kolloidales Silber freisetzt. Dies gilt sowohl für die äußere Beschichtung des Schlauches als auch seine innere Beschichtung. Techniken zum Aufbringen von derartigen Beschichtungen sind als solche aus anderen Bereichen bekannt, zum Beispiel aus der allgemeinen Technik von Metallbeschichtungen.

Die Konzentration und/oder Schichtstärke der antiseptisch oder antimikrobiell wirkenden, Metallionen oder kolloidales Silber freisetzenden Substanzen ist vorzugsweise so bemessen, dass sie insbesondere in den ersten 20 Tagen des Einsatzes wirken, solange die Infektionsgefahr besteht.

Die hier beschriebenen Schläuche bestehen vorzugsweise aus Polyurethan oder Silikon. Es können auch mehrschichtige Schläuche vorgesehen werden, zum Beispiel zweischichtige mit einer etwas dünneren Außenschicht, in welcher oder auf welcher die antiseptisch oder antimikrobiell wirkenden Substanzen angeordnet sind, während eine vorzugsweise etwas stärkere innere Schlauchschicht davon frei sein kann. Eine solche mehrschichtige Schlauchkonstruktion ist im Extrusionsverfahren leicht herstellbar. Die Schichtanordnung kann auch umgekehrt als vorstehend beschrieben sein.

Die Erfindung lehrt auch allgemein als antiseptisch oder antimikrobiell wirkende, Metallionen freisetzende Substanz die Verwendung von ionisiertem Titan, d.h. einer Verbindung, welche Titan in ionischer Form freisetzt. Diese Verwendung bezieht sich insbesondere auf PEG-Schläuche und allgemein Schläuche für die enterale Ernährung.

Nachfolgend wird ein Ausführungsbeispiel der Erfindung anhand der Zeichnung näher erläutert.

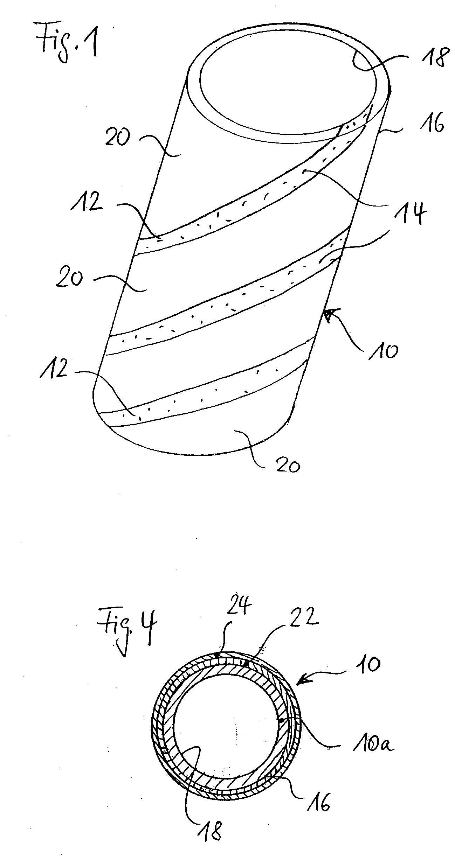

Die Figuren 1, 2 und 3 zeigen schematisch jeweils einen Abschnitt eines PEG-Schlauches. Figur 4 zeigt einen Schnitt durch ein Ausführungsbeispiel eines Schlauches für die enterale Ernährung.

In den Figuren sind einander entsprechende oder funktionsähnliche Komponenten mit den gleichen Bezugszeichen versehen.

Figuren 1, 2 und 3 zeigen einen kurzen Abschnitt eines PEG-Schlauches 10 aus PU. In ausgewählten Bereichen 12 des Schlauches sind Silber-Nano-Partikel 14 eingebracht. Die dazwischenliegenden Bereiche 20 sind frei von solchen Partikeln.

Die mit den silberhaltigen Nanopartikeln versehenen Bereiche 12 erstrecken sich von der Außenfläche 16 des Schlauches 10 bis zur Innenfläche 18.

Die Konzentration der silberhaltigen Nanopartikel ist so, dass sie über längere Zeiträume, insbesondere Zeiträume von vielen Tagen, Wochen oder Monaten kontinuierlich Silberionen mit antibakterieller Wirkung abgeben.

Figur 1 zeigt eine Anordnung der mit der antibakteriellen Verbindung versetzten Bereiche 12, die sich wendelförmig (schraubenlinienförmig) um die Längsachse des Schlauches 10 herum erstrecken.

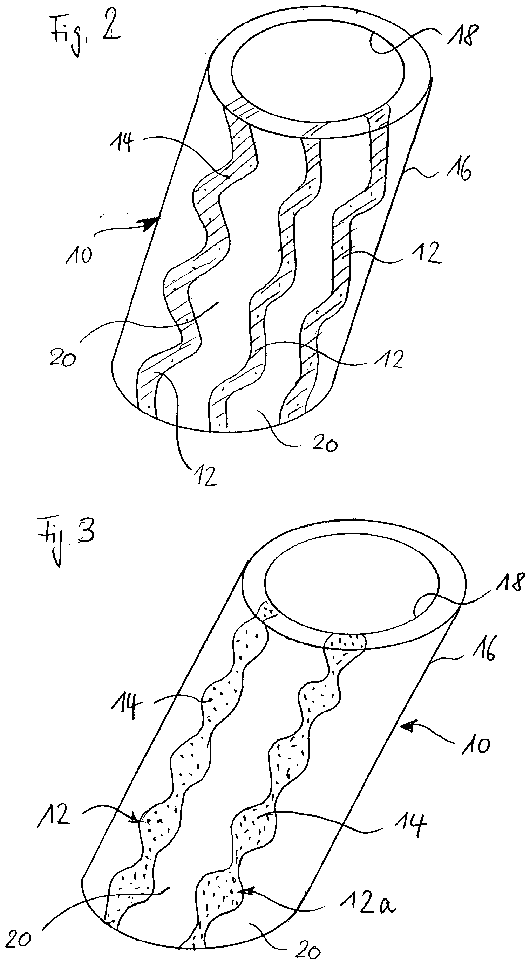

Gemäß Figur 2 erstrecken sich die mit der antibakteriellen Verbindung versetzten Bereiche 12 in Längsrichtung des Schlauches und sind zick-zack-förmig bzw. wellenförmig.

Gemäß Figur 3 sind die entsprechenden Bereiche 12 so gestaltet, dass ihre Breite in Längsrichtung des Schlauches variiert.

Die Darstellungen sind schematisch. Tatsächlich sind die mit der antibakteriellen Substanz versetzten Bereiche so geometrisch gestaltet, dass die wirksamen Metallionen weitestgehend homogen vom Schlauch abgegeben werden, d.h. die verbindungstragenden Bereiche sind so eng beieinanderliegend, dass keine Lücken hinsichtlich der antimikrobiellen Wirkung verbleiben.

Der Schlauch 10 ist mit einer Beschichtung versehen, die das Eindringen eines Polyvidon-Jod-Komplex-Wirkstoffes und/oder das Eindringen von Desinfektionsmittel in das Schlauchmaterial verhindert. Für diese Beschichtung ist besonders Parylene geeignet.

Die Innenfläche 18 des Schlauches ist mit einer Schicht versehen, vorzugsweise im Koextrusionsverfahren, die resistent ist gegen Zytostatika, wodurch der Schlauch seine mechanischen Eigenschaften beibehält. Insbesondere bietet Parylene Schutz gegen Zytostatika.

Als Beschichtungsmaterial für den Schlauch kommt insbesondere Parylene oder Poly-Parylene (Handelsname) in Betracht. Parylene ist ein Polymer aus der Familien Polyp-Xylylene.

Figur 4 zeigt einen Schnitt durch ein Ausführungsbeispiel eines Schlauches 10 für die enterale Ernährung, insbesondere für die PEG. Ein Schlauch 10a aus Polyurethan oder Silikon bildet das Stützgerüst des Schlauches. Auf den Schlauch 10a aufgetragen ist radial außen eine Schicht 22 aus Parylene, insbesondere Parylene C oder Parylene N oder einem Mix aus Parylene C und Parylene N. Über die Schicht 22 ist eine weitere Schicht 24 aufgebracht aus einem antimikrobiell wirkenden, Metallionen freisetzenden Material, wobei die Metallionen bevorzugt Silberionen oder Titanionen sind. Auch kann statt der Schicht 24 oder zusätzlich dazu eine kolloidales Silber oder ein anderes antimikrobiell wirkendes Kolloidales Metall freisetzende Schicht vorgesehen sein.

Das Ausführungsbeispiel gemäß Figur 4 kann dahingehend ergänzt werden, dass eine analoge Beschichtung auf der Innenfläche 18 des Schlauches 10a aufgebracht wird, also auf die Innenfläche 18 zunächst eine Beschichtung (analog der Schicht 22) aus Parylene C und/oder Parylene N und innenseitig darauf dann eine weitere Beschichtung (analog der Beschichtung 24) aus einem Metallionen und/oder kolloidales Metall freisetzenden Material.

In Figur 4 sind die Schichtstärken nicht maßstabsgetreu; vielmehr sind zur vereinfachten Darstellung die äußeren Schichten verstärkt gezeichnet.

In der vorstehenden Beschreibung und den Ansprüchen sowie der Zeichnung sind allgemein Schläuche für die enterale Ernährung dargestellt, insbesondere PEG-Schläuche. Sämtliche beschriebenen Einzelheiten, Merkmale und Maßnahmen sind sowohl für den Einsatz bei PEG-Schläuchen als auch ganz allgemein für den Einsatz bei Schläuchen für die enterale Ernährung geeignet und einsetzbar.

## Patentansprüche

1. PEG-Schlauch mit einer antiseptisch oder antimikrobiell wirkenden, Metallionen oder kolloidales Metall freisetzenden Verbindung, **dadurch gekennzeichnet, dass** die genannte Verbindung in Form von Nanopartikeln vorliegt und in Bereichen des Schlauches (10) homogen im Material des Schlauches (10) über dessen Stärke verteilt ist.

2. PEG-Schlauch nach Anspruch 1, **dadurch gekennzeichnet, dass** der Schlauch (10) eine Parylene-Beschichtung aufweist, insbesondere aus Parylene N und/oder Parylene C.

3. PEG-Schlauch nach Anspruch 2, **dadurch gekennzeichnet, dass** in die Parylene-Beschichtung eine antimikrobiell wirkende, Metallionen freisetzende Verbindung eingemischt ist, oder dass auf die Parylene-Beschichtung eine Schicht (24) aus einem Metallionen, insbesondere Silber- oder Titan-Ionen, freisetzenden Material aufgebracht ist.

4. PEG-Schlauch nach Anspruch 2 oder 3, **gekennzeichnet durch** eine Parylene-Beschichtung auf der Außenfläche (16) und/oder Innenfläche (18) des Schlauches (10).

5. PEG-Schlauch nach Anspruch 1, **dadurch gekennzeichnet, dass** die Bereiche (12) sich streifenförmig in Längsrichtung des Schlauches (10) erstrecken.

6. PEG-Schlauch nach Anspruch 5, **dadurch gekennzeichnet, dass** die streifenförmigen Bereiche (12) zick-zack-förmig, wellenförmig oder wendelförmig sind.

7. PEG-Schlauch nach Anspruch 6, **dadurch gekennzeichnet, dass** die streifenförmigen Bereiche (12a) eine variierende Breite haben.

8. PEG-Schlauch nach einem der vorhergehenden Ansprüche, **dadurch gekennzeichnet, dass** der Schlauch (10) zumindest teilweise transparent ist.

9. PEG-Schlauch nach einem der vorhergehenden Ansprüche, **dadurch gekennzeichnet, dass** der Schlauch (10) zumindest teilweise opak ist.

10. PEG-Schlauch nach einem der vorhergehenden Ansprüche aus Polyurethan oder Silikon.

## Claims

1. A PEG tube containing an antiseptically or antimicrobially acting compound that releases metal ions or colloidal metal, **characterized in that** the said compound is present in the form of nanoparticles, and in regions of the tube (10) is homogeneously distributed in the material of the tube (10) over the thickness thereof.

2. The PEG tube according to Claim 1, **characterized in that** the tube (10) has a Parylene coating, in particular made of Parylene N and/or Parylene C.

3. The PEG tube according to Claim 2, **characterized in that** an antimicrobially acting compound that releases metal ions is mixed into the Parylene coating, or that a layer (24) made of a material that releases metal ions, in particular silver or titanium ions, is applied to the Parylene coating.

4. The PEG tube according to Claim 2 or 3, **characterized by** a Parylene coating on the outer surface (16) and/or inner surface (18) of the tube (10).

5. The PEG tube according to Claim 1, **characterized in that** the regions (12) extend in a strip shape in the longitudinal direction of the tube (10).

6. The PEG tube according to Claim 5, **characterized in that** the strip-shaped regions (12) have a zig-zag, undulating, or helical shape.

7. The PEG tube according to Claim 6, **characterized in that** the strip-shaped regions (12a) have a varying width.

8. The PEG tube according to one of the preceding claims, **characterized in that** the tube (10) is at least partially transparent.

9. The PEG tube according to one of the preceding claims, **characterized in that** the tube (10) is at least partially opaque.

10. The PEG tube according to one of the preceding claims, made of polyurethane or silicone.

## Revendications

1. Tubulure de PEG comprenant un composé antiseptique ou antimicrobien libérant des ions métalliques ou un métal colloïdal, **caractérisée en ce que** ledit composé est présent sous forme de nanoparticules et réparti de façon homogène dans certaines zones de la tubulure (10) dans le matériau de la tubulure (10) à travers l'épaisseur de celle-ci.

2. Tubulure de PEG selon la revendication 1, **caractérisée en ce que** la tubulure (10) présente un revêtement de parylène, en particulier de parylène N et/ou de parylène C.

3. Tubulure de PEG selon la revendication 2, **caractérisée en ce qu'**un composé antimicrobien libérant des ions métalliques est mélangé au revêtement de parylène ou **en ce qu'**une couche (24) d'un matériau libérant des ions métalliques, en particulier des ions d'argent ou de titane, est appliquée au revêtement de parylène.

4. Tubulure de PEG selon les revendications 2 ou 3, **caractérisée par** un revêtement de parylène sur la surface extérieure (16) et/ou la surface intérieure (18) de la tubulure (10).

5. Tubulure de PEG selon la revendication 1, **caractérisée en ce que** les zones (12) s'étendent sous forme de bandes dans la direction longitudinale de la tubulure (10).

6. Tubulure de PEG selon la revendication 5, **caractérisée en ce que** les zones (12) en forme de bandes sont en forme de zigzag, ondulées ou hélicoïdales.

7. Tubulure de PEG selon la revendication 6, **caractérisée en ce que** les zones (12a) en forme de bande présentent une largeur variable.

8. Tubulure de PEG selon l'une quelconque des revendications précédentes, **caractérisée en ce que** la tubulure (10) est au moins partiellement transparente.

9. Tubulure de PEG selon l'une quelconque des revendications précédentes, **caractérisée en ce que** la tubulure (10) est au moins partiellement opaque.

10. Tubulure de PEG selon l'une quelconque des revendications précédentes en polyuréthane ou silicone.
